# EUROPEAN PATENT APPLICATION

(11) **EP 1 566 142 A1**
(43) Date of publication of application: **24.08.2005**
(21) Application number: 04075541.5
(22) Date of filing: 19.02.2004
(51) Int. Cl.: A61B 5/00, G01N 21/47

(54) **Imaging of buried structures**

(71) Applicant: NEDERLANDSE ORGANISATIE VOOR TOEGEPAST-NATUURWETENSCHAPPELIJK ONDERZOEK TNO, 2628 VK Delft (NL)
(72) Inventor: Wieringa, Fokko Pieter, 6921 JA Duiven (NL); Bakker, Dirkjan, 2403 VN Alphen aan den Rijn (NL); van der Steen, Antonius Franciscus Wilhelmus, 3043 BD Rotterdam (NL); Mastik, Frits, 3011 RC Rottderdam (NL); van Melick, Rene Gerardus Maria, 1156 BH Marken (NL)
(74) Representative: Prins, Adrianus Willem

(57) **Abstract**

A method of obtaining an image of buried structures in an object, comprising partially irradiating said object by light of a wavelength that images said buried structure; and obtaining a complementary partial image of said buried structure by light diffused from a non-irradiated image area.

According to the method, an image can be obtained while discarding specular reflections of the object. Thus, deeper structures will be better visible. In a further aspect, the invention offers a method of enhancing imaging of buried structures in an object, comprising: analysing said image as a first image in order to detect the edges of said buried structure; obtaining a second image by irradiating said object with a second light that visually images said object; and combining said first and second images for defining edges of said buried structure in said visual image.

## Description

The invention relates to a method of obtaining an image of buried structures in an object, in particular to imaging structures such as vascular structures in biological tissue by means of infra-red light.

A method describing such is disclosed in the international application WO0115597 by the same inventor. It has been found, that obtaining a sufficiently clear image is difficult due to various problems. One problem is, that visual light emanating from buried, in particular deeper parts of the object is often much weaker than the light that is reflected directly by the surface of the object. In practice, this means that a separation of the specularly reflected light and light emerging from deeper parts of the object may be needed in order to identify underlying structures.

While separating these two types of light, for instance by a known method of using polarized light and using the fact that specularly reflected light keeps its polarization direction, so that it can be filtered out by means of a polarizing filter, a substantial amount of the light is lost that is originating from the lower parts of the object, thus resulting in a loss of image brightness and resolution. This invites to the use of powerful light sources in order to receive sufficient light from the lower parts in the process of separating the two parts. However, especially in the area of imaging structures in live objects, that there is a maximum amount of light that may be irradiated on the object.

The invention has as an object to provide an imaging technique that does not suffer from the afore described problems and that is able to provide an enhanced image of the underlying structure. Moreover, the invention has as an object to provide an imaging enhancement technique to enable a person to combine visual information and information of buried objects in one image.

To achieve these and other goals, in one aspect, the invention offers a method according to the features of claim 1. In another aspect, the invention offers a method according to the features of claim 18.

In particular, by partially irradiating said object by light of a wavelength that images said buried structure; and obtaining a complementary partial image of said buried structure by light diffused from a non-irradiated image area, light incident on the image that is originating from a direct reflection is spatially filtered out of the image. The remaining partial image does not suffer from saturation effects due to direct illumination of specularly reflected light. In a preferred embodiment, a full image is provided by varying said partial irradiation in time so as to provide a full image by subsequent combining of said partial images.

In a further preferred embodiment, said partial image is obtained by scanning a light beam over said object. In addition or alternatively, said partial image is obtained by subsequently irradiating said object by predetermined patterns. One particularly preferred embodiment comprises obtaining said partial image by alternatingly irradiating said object by a predetermined complementary patterns. For instance, in an embodiment said patterns may be matrix-patterns, line patterns or circular patterns.

Further, preferably said object is irradiated only at predetermined positions that are spaced apart. By spacing the irradiation area and the light detection area, deeper parts of the buried structure may be enhanced.

By alternatively illuminating said object, a full image may be provided, and wherein all areas of the object are irradiated in a time-dependent manner. It has been found that a particularly preferential embodiment is an embodiment, wherein said image is obtained by a pixelmatrix of CCD's, and wherein said partial image is obtained by discarding pixels measuring a light intensity that is higher than a predetermined light intensity. This embodiment provides a simple implementation of how the image analysis can be performed without complicated logic. Just by discarding "overexposed" or "saturated" pixels the remainder of the image can be retained and combined with subsequently received partial images. Alternatively, or in addition, said image is obtained by a pixelmatrix of CCD's, and wherein said partial image is obtained by discarding pixels that are expected to be irradiated by non-diffuse light. Said expectance may be based on a time-variation of said partial irradiation. Thus, by synchronizing the time-variation of said partial irradiation and the reception of light on said camera-pixels (in anti-phase), pixels may be discarded from the image that receive light that is originating from a direct specular reflection on the surface of the object.

The invention further offers particular benefits while using a CMOS-camera as a pixel matrix, since these camera's have a high degree of decorrelation of adjacent pixels. Thus, the effect of "blooming" is prevented, so that there is a high contrast between the directly reflected area (that is discarded) and the remaining area which receives diffuse light originating from deeper layers.

Furthermore, the invention preferably is used while analysing said image as a first image in order to detect the edges of said buried structure; obtaining a second image by irradiating said object with a second light that visually images said object; and combining said first and second images for defining edges of said buried structure in said visual image. This offers the benefit of obtaining a "normal" visual image, that is enhanced by identifying the underlying structure in the visual image. For instance, for surgical purposes, the method offers a convenient tool for deciding an optimal entry point in the object, for instance for cutting tissue or the like.

Preferably, said edge-detection is performed by a gradient analysis of said first image. Further, preferably, said first and second images each comprise a pixel matrix that is multiplied numerically on a pixel-by pixel basis. In this way, edges, represented by black lines (having a low intensity number) will appear in the visual image by corresponding lowering the visual intensity level by multiplication of the black level from the first image.

The invention offers a convenient embodiment when said images are provided stereoscopically. Furthermore, said first image may be spectrally analysed, and wherein said spectral analysis is projected into said second image. Furthermore, said spectral analysis may comprise a pulsatility analysis and/or a hart beat frequency analysis and/or respiratory frequency analysis. Such analysis thus offers a convenient non-contact tool for measuring body parameters of interest. Under "pulsatility analysis" is understood at least a determination of pulsating parts in the object of interest.

Further features and benefits will become apparent from the figures. In the figures:
Figure 1 shows a schematic view of a scanning irradiating method of an object according to the invention;
Figure 2 shows a schematic view of foton-migration in live tissue due to diffusion;
Figure 3 shows an illumination method by illuminating an overlapping pattern on the object;
Figure 4 shows an illumination method by illuminating a circular patterns on the object;
Figure 5 shows an illumination method by illuminating a grid of patterns on the object;
Figure 6 shows a testing arrangement for testing the apparatus according to the invention;
Figure 7 shows a series of analysis steps for processing and routing of data acquired from the arrangement of Figure 6;
Figure 8 shows a spectral analysis of the pulsatile components in said image for light of three different wavelengths;
Figure 9 shows an a visual image that is enhanced by combining images for defining edges of said buried structure in a visual image;
Figure 10 shows a simple vizor-like construction comprising the apparatus of the invention; and
Figure 11 shows a the pulsatility in a part of a human arm.

Imaging deeper structures by polarization filters are known from the prior art. The disadvantage of polarization filters is that even an ideal polarization filter in principle causes a transmittance loss of 50%. Still we describe here how our device would look like when using this method (applies to single configuration AND stereoscopic configuration): ringlights are equipped with an optical polarization filter from which the orientation can be rotated. The rotational orientation of both ringlight polarization filters is kept parallel by a first coupling mechanism.

Camera's are also equipped with an optical polarization filter with variable rotational orientation. Also here the rotational orientation of both camera polarization filters is kept parallel by a second coupling mechanism. The rotational angular difference ϕ between the ringlight polarization and the camera polarization can be set and adjusted between 0 and 90 degrees.

Two modes of rotation adjustments can be distinguished:
A. Surface polarization adjustment
   Here the ringlight polarization can be varied to minimize surface glare whilst the rotational angular difference ϕ between ringlight polarization and camera polarization is kept constant.
B. Specular / diffuse reflection adjustment
   Specular reflections (mainly occurring at the surface) can be increasingly suppressed by adjusting the rotational angular difference ϕ from 0 to 90 degrees. This relatively enhances the more diffuse reflections from deeper within the tissue. Since the skin is the first encountered reflective surface, it produces a much higher specular reflectance component than more deeper tissue structures. Bloodvessels are located underneath the skin, the image contrast provided by the bloodvessels is highly diffuse.

In an embodiment, the camera polarization filters can also be made on/off switchable. Using a high framerate, images then can be captured with the camera polarization filters switched on respectively off, thus allowing additional image enhancement by electronic subtraction.

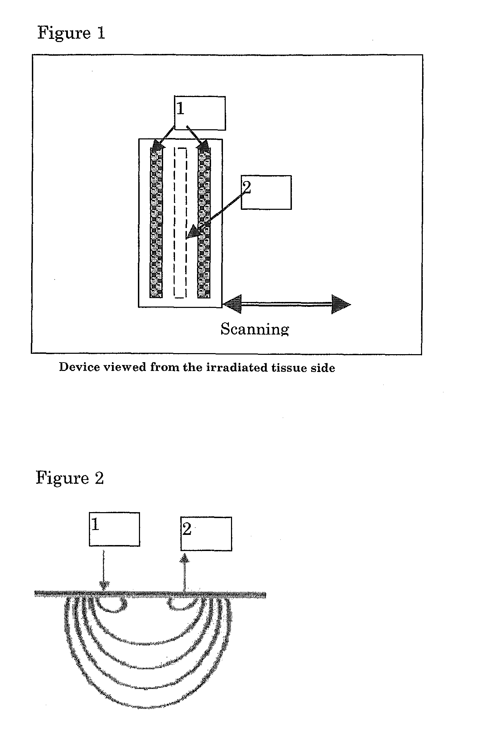

Figure 1 shows an alternative to the above described polarization filtering method. This method comprises dynamic complementary lighting/scanning of alternating patterned image sections. This method does not require the use of polarization filters. It is based on the fact that photons entering biological tissue will strongly scatter within the tissue which partly results in backscattering.

Furthermore the viewed area is divided into parallel linear areas , which we will call "line sections". These line sections can be divided into even and uneven line sections 1, 2 respectively.

Using a camera that has good anti-blooming specifications and allows the read-out of freely selectable rectangular pixel regions we then can acquire image information in a special sequence.

During a certain period the specially constructed light source will light all even line sections 1 and the camera will acquire image information from all uneven line sections 2. During the next period the uneven line sections 2 are lighted and the camera will acquire image information from the even line sections. This can either be done with a line camera that scans the entire tissue or with a normal camera that scans all even lines simultaneously and during the next period all uneven lines.

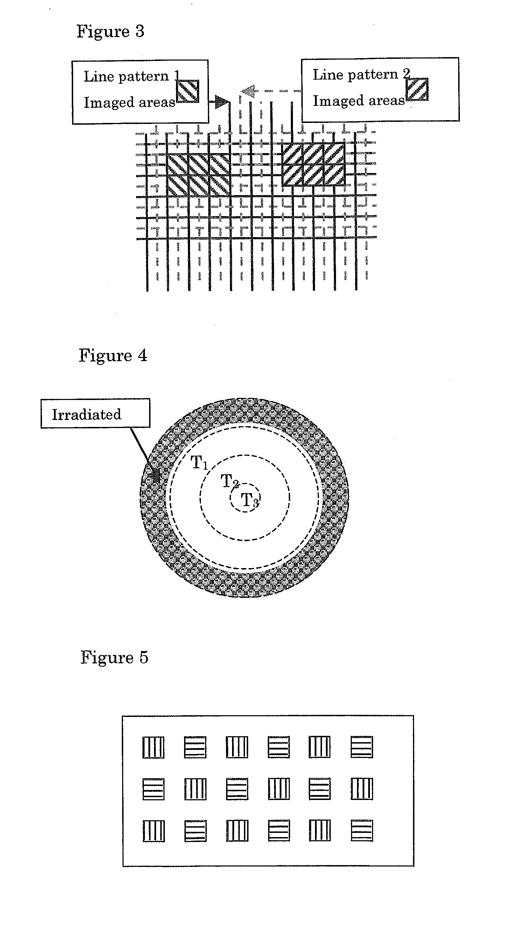

In Figure 2 is illustrated how diffuse light can be used to image deeper parts of the object. Light enters the object at one position 1 and leaves the object at another position 2. From the figure it becomes clear that deeper parts of the object enter the object at further distanced positions. By applying a variety of illumination patterns as will be further described with reference to Figure 3 - Figure 5, illumination from "within" the object can be achieved, thus imaging deeper parts of the object.

To this end, in Figure 3, instead of even and uneven parallel lines shown in Figure 1, alternate spatially shifted crossed line patterns can be used as lighting pattern whilst image acquisition occurs within the areas between the lines. Also, in Figure 4 discrete concentric circular areas can be used. Satisfying results were obtained by a simple test of circular irradiation geometry disclosed in Figure 4. A 6mm thick slice of pink foam (3M ethafoam) was laid upon the shielding pipe. On top of this foam slice a plastic office clamp was placed, with its' white plastic paper retaining slice laying across it. Finally a second slice of pink foam was laid on top. Outside the camera's field of view, the ring light injected photons of 660 nm, 810 nm and 940 nm into the foam, perpendicular to the foam surface.

Figure 5 shows another embodiment, wherein said object is irradiated only at predetermined positions that are spaced apart. First areas 3 indicated with horizontal lines are irradiated in a first period; second areas 4 with vertical lines are irradiated in a second period. Such a spaced apart configuration is able to show deeper parts of the structure. By varying the spacing, lower and deeper parts of the object may be scanned.

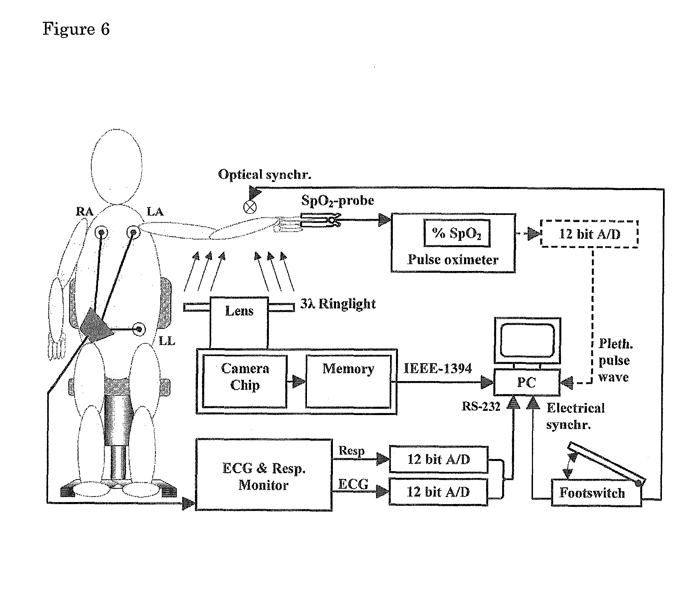

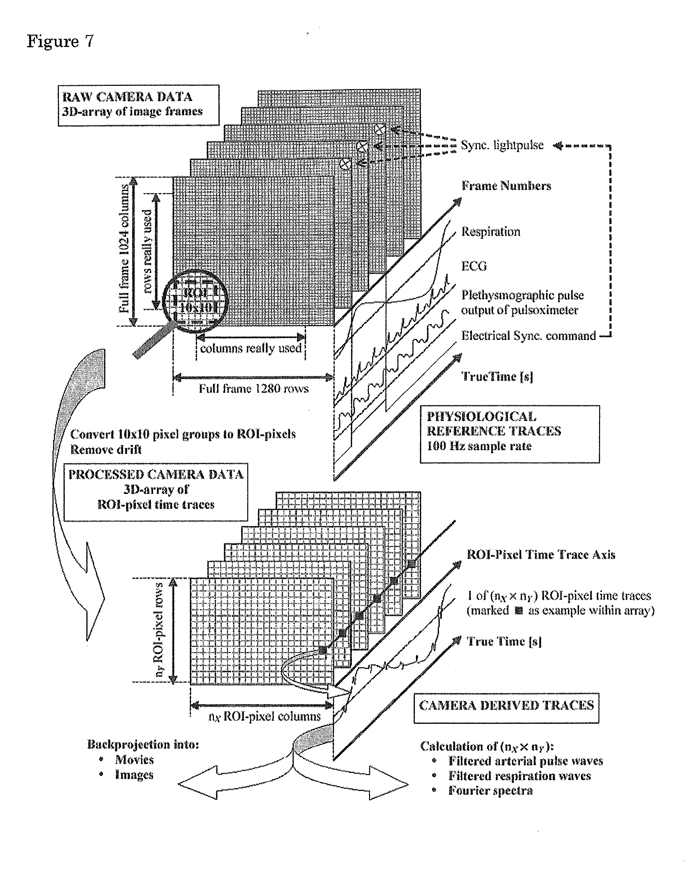

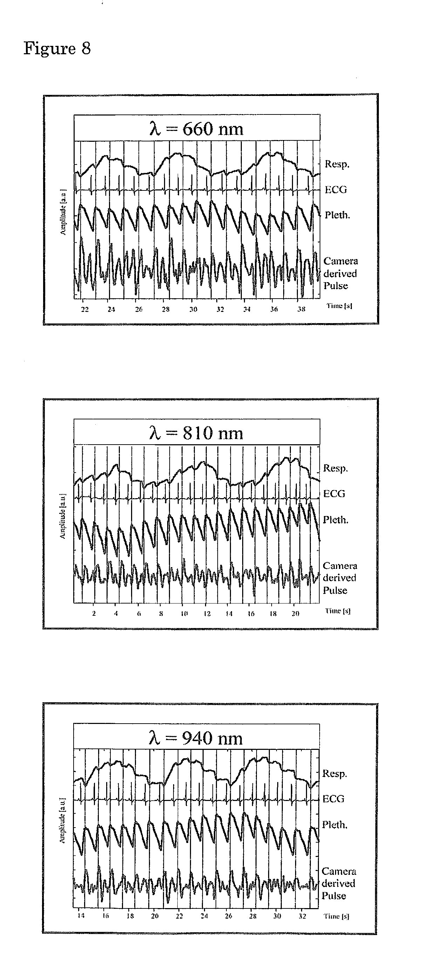

Figure 6 shows a testing arrangement, where the method of the invention was tested using known markers such as an SP02 pulse oximeter, an ECG recording devise and a respiratory frequency monitor. The signals were recorded and sampled using the steps indicated in Figure 7. This leads to a pixel by pixel time-analysis of intensity variation. The frames were sampled at a 100Hz Sample rate and the recorded respiration, ECG and plethismographic pulse output were compared. The outcome is illustrated for a variety of wavelengths in Figure 8. It is clearly shown how well the measured variation of the camera matches with the other pulse signals.

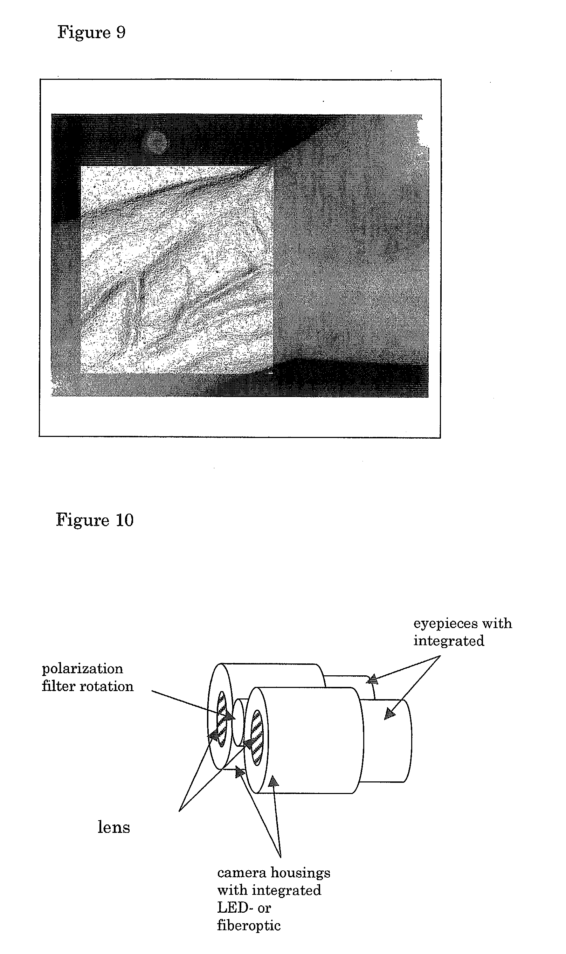

Figure 9 shows an image of a human member wherein the signals of an image of deeper vascular structures are combined with a visual image. Such an image may be of use for better guiding a physician in handling the patient.

The invention may be used by a camera wherein the spectral response capable of imaging within the visual range (400 - 780nm) is extended so that at least 1 spectral band outside the visible range can additionally be captured. The response band(s) outside the visible range band may be located within the ultraviolet range (100 - 400nm) or within the near infrared range (780 - 1500nm). The camera is connected to an image processing device and a display device.

By using different modes of the processing device the user can either:
A. View only the normal image as aquired within the visible range; or
B. View only the additional image(s) aquired outside the visible range; or
C. View an image as aquired within the visible range with freely user adjustable area within the displayed visible image in which a image aquired outside the visible range is projected (as a grey scale or in case of a multiband as a false colour image); or
D. View an image as aquired within the visible range with a superimposed backprojection of a image aquired outside the visible range that has been subjected to processing.

This backprojection can be achieved by embossing a grey-scale processed image (that was aquired outside the visual range) upon a colour image by multiplying the brightness value of each colour pixel with the grey-scale value of each corresponding "non-visible range" pixel. An example of such a back projection is illustrated in Figure 9.

Although a raw image (left picture) of the same tissue (here acquired at 940nm) contains much more grey levels, edge enhancement enhances recognition of the vessel network structure (right picture). The image on the right contains mainly information about the vessel structure. This Black & White information can be backprojected on a (colour) image that represents the normal visible range. In case that more than 1 wavelength band is acquired outside the visible range, the result may also be displayed as a false colour image.

A special configuration with added value is based upon two cameras (e.g. CCD or CMOS monochromatic or multiband) positioned at a certain distance from each other (e.g. eye-to-eye distance) thus stereoscopically viewing the same object (e.g. biological tissue), a dual channel electronic image processing device and two display devices placed in front of both eyes.

Between each camera and the viewed object an additional optical system (maybe combining 2 channels) may be placed (e.g., a dual channel microscope, endoscope, colposcope, etc.) It is possible to incorporate a simple vizor-like construction (see Figure 10) so that the device can be either put in front of the eye or be positioned out of the viewing angle to allow normal sight. It is possible to apply cameras capable of imaging in two modes:
1. Visible colour imaging
2. Monochrome (low cost) or multiband false colour infrared imaging (higher cost).

Each of the two camera may be equipped with:
1. a ringlight capable of emitting at least one band of optical radiation outside the visible range. The camera may be equipped with a multiband ringlight.
2. one or more optical bandpass filter(s) tuned to the ringlight wavelength(s).

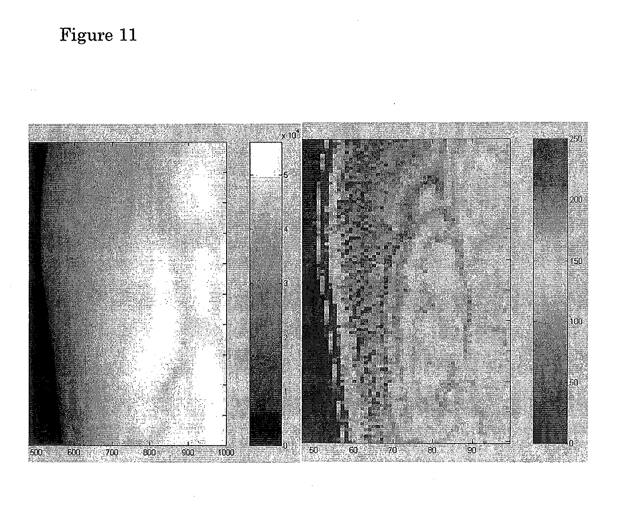

Figure 11 shows a visual image of a part of a human arm (left), where (left) the pulsatility is shown in greyed values. It is clear that this technique offers promising views not only to show buried structures, but also to detect pulsatile properties thereof.

The invention is further characterized by the following features and benefits: Imaging of anatomical structures:
1. A contactless imaging device for improved visualisation of blood vessels or other contrasting structures for medical diagnosis and/or assistance during surgery, insertion of intravasal devices (e.g. a venflon or a needle during blood withdrawal).
2. A stereoscopic form offers improved eye to hand co-ordination (better judgement of 3D-structures) which is beneficial for the previously mentioned application.
   Haemodynamical imaging:
3. Using a device according to [1] or [2] it is possible to produce images of a pigment spreading into the body. Such a pigment may be either injected into the bloodstream as a fluid (e.g. Indocyanine green or methylene blue) or administered by respiration of a tracer gas (e.g. Carbon Monoxide which is used in lung function measurements or Nitrogen monoxide which is used as a vasal dilatation drug).
   General favourable aspects:
4. By choosing an appropriate wavelength for a device according to [1] [2] or [3] the influence of skin pigmentation can be eliminated as illustrated on previously shown images (acquired with a simple setup).
5. For a device according to [1] [2] [3] or [4] the contrast of blood soaked sterile materials like bandages is improved within the near infrared so that such objects can be located easier during surgery (thus decreasing the chance that they are left inside a patient when the wound is closed).
   Deriving physiological signals (heart rate & respiration)
6. Derivation of a 2-dimensional image of pixel groups having each a cardiac pulse (described in our previous patent application WO 01/15597 A1) from images acquired by a device according to [1] or [2] is possible.
7. Additional derivation, likewise to [6], of a respiration correlated photoplethysmographic pulse has also been demonstrated.
8. Derivation of perfusion related parameters can be performed on signals delivered by a device according to [3].
   Improved penetration of structures buried within the tissue is possible by several approaches:
9. For all previous features from [1] to [8]: Application of different polarization of the projected light with regard to the opening.
10. For all previous features s from [1] to [8]: Alternately activated geometrically shifted patterns of optical radiation (e.g., parallel lines, a crossed line mesh or concentric circles) can be projected onto the tissue. The directly irradiated image portions can be automatically identified. This utilizes the identification of the tissue portions that are not directly irradiated but mainly emit photons that have entered the tissue and are scattered back towards the camera(s). The full image can then be reconstructed from the different acquired portions of the viewed tissue.

## Claims

1. A method of obtaining an image of buried structures in an object, comprising:
- partially irradiating said object by light of a wavelength that images said buried structure; and
- obtaining a complementary partial image of said buried structure by light diffused from a non-irradiated image area.

2. A method according to claim 1 wherein said partial irradiation is varied in time so as to provide a full image by subsequent combining of said partial images.

3. A method according to claim 1 or 2, wherein said partial image is obtained by scanning a light beam over said object.

4. A method according to claim 1 or 2, wherein said partial image is obtained by subsequently irradiating said object by predetermined patterns.

5. A method according to claim 4, wherein said partial image is obtained by alternatingly irradiating said object by a predetermined complementary patterns.

6. A method according to claim 4 or 5, wherein said patterns are matrix-patterns, line patterns or circular patterns.

7. A method according to any of claims 4-6, wherein said object is irradiated only at predetermined positions that are spaced apart.

8. A method according to any of claims 1-7, wherein said image is obtained by a pixelmatrix of CCD's, and wherein said partial image is obtained by discarding pixels measuring a light intensity that is higher than a predetermined light intensity.

9. A method according to any of claims 1-8, wherein said image is obtained by a pixelmatrix of CCD's, and wherein said partial image is obtained by discarding pixels that are expected to be irradiated by non-diffuse light.

10. A method according to claim 9 wherein said expectance is based on a time-variation of said partial irradiation

11. A method according to claim 9-10 wherein said pixel matrix is a CMOS-camera.

12. A method according to any of the preceding claims, further comprising:
- analysing said image as a first image in order to detect the edges of said buried structure;
- obtaining a second image by irradiating said object with a second light that visually images said object; and
- combining said first and second images for defining edges of said buried structure in said visual image.

13. A method according to any of the preceding claims, wherein said edge-detection is performed by a gradient analysis of said first image.

14. A method according to claim 12 or 13, wherein said first and second images each comprise a pixel matrix that is multiplied numerically on a pixel-by pixel basis.

15. A method according to any of the preceding claims, wherein said images are provided stereoscopically.

16. A method according to any of the preceding claims 12- 15, wherein said first image is spectrally analysed, and wherein said spectral analysis is projected into said second image.

17. A method according to claim 16, wherein said spectral analysis comprises a pulsatility analysis and/or a hart beat frequency analysis and/or respiratory frequency analysis.

18. A method of enhancing imaging of buried structures in an object, comprising:
- obtaining a first image by irradiating said object with a first light of a wavelength that images said buried structure;
- analysing said first image in order to detect the edges of said buried structure;
- obtaining a second image by irradiating said object with a second light that visually images said object; and
- combining said first and second images for defining edges of said buried structure in said visual image.

19. A method according to claim 18, wherein said first image is obtained by separating polarized reflected light from diffuse light.

20. Apparatus for obtaining an image of buried structures in an object, comprising:
- an irradiating device for partially irradiating said object by light of a wavelength that images said buried structure; and
- a camera device for obtaining a complementary partial image of said buried structure by light diffused from a non-irradiated image area; and
- a control device for varying said irradiation device in time and
- a processing device for acquiring a full image by subsequent combining of said partial images.

21. An apparatus according to claim 20 wherein wherein said camera is arranged for obtaining a second image by irradiating said object with a second light that visually images said object; and wherein said processing device is arranged for detecting the edges of said buried structure by image analysis; and for combining said first and second images for defining edges of said buried structure in said visual image.
